# EUROPÄISCHE PATENTSCHRIFT

(11) **EP 2 300 176 B1**
(45) Veröffentlichungstag und Bekanntmachung des Hinweises auf die Patenterteilung: **21.12.2011**
(21) Anmeldenummer: 09780028.8
(22) Anmeldetag: 30.06.2009
(51) Int. Cl.: B08B 7/00

(54) **VORRICHTUNG ZUR REINIGUNG VON GEGENSTÄNDEN**
DEVICE FOR CLEANING OBJECTS
SYSTÈME POUR NETTOYER DES OBJETS

(30) Priorität: 02.07.2008 DE 202008008729 U; 23.12.2008 DE 102008063050
(43) Veröffentlichungstag der Anmeldung: 30.03.2011
(73) Patentinhaber: Reinhausen Plasma GmbH, 93057 Regensburg (DE)
(72) Erfinder: BISGES, Michael, 93161 Sinzing (DE); KRÜGER, Thorsten, 93049 Regensburg (DE); WICHMANN, Patrick, 32469 Petershagen (DE); ARNING, Hans-Jürgen, 32312 Lübbecke (DE)
(74) Vertreter: Kohlmann, Kai
(86) Internationale Anmeldenummer: PCT/EP2009/058184
(87) Internationale Veröffentlichungsnummer: WO 2010/006921

(56) Entgegenhaltungen:
- DE-A1- 10 321 889
- US-A- 5 236 512
- US-A1- 2006 144 427
- US-A1- 2008 073 595

## Beschreibung

Die vorliegende Erfindung betrifft eine Vorrichtung zur Reinigung von Gegenständen, insbesondere von Matratzen.

Es sind Staubsauger bekannt, mittels denen eine Reinigung von Teppichen, Polstermöbeln und Matratzen möglich ist. Dabei kann ein Staubsauger allerdings keine Gerüche neutralisieren und auch Keime und Sporen lassen sich nur schlecht absaugen und bleiben daher im Textil erhalten.

Ferner gibt es Nassreiniger, die eine gewisse Entkeimungswirkung besitzen. Solche Nassreiniger wirken aber nur oberflächlich und benötigen eine lange Trocknungszeit. Die Reinigung ist vergleichsweise aufwendig und es muss darauf geachtet werden, dass nicht zuviel Nässe in das Textil eindringt.

Zudem gibt es UV-Lampen, die oberflächlich wirksam sind und dort Keime und Sporen abtöten. Es ist allerdings nicht möglich, einen Bereich innerhalb des Textils damit zu erreichen, so dass die Wirksamkeit sehr begrenzt ist.

Aus der US 5 236 512 A ist eine Vorrichtung zur Reinigung von Oberflächen bekannt, welche eine Druckgasquelle umfasst, die über eine Rohrleitung mit einem Reinigungskopf verbunden ist. Der Reinigungskopf ist über die zu reinigende Oberfläche bewegbar. In einer Reaktionskammer des Reinigungskopfes sind zwei Elektroden angeordnet, die mit einer Spannungsquelle verbunden sind. Eine elektrische Entladung zwischen den Elektroden erzeugt in der Reaktionskammer reaktive Gase, unter anderem Plasma. Unter Einwirkung der reaktiven Gase lassen sich Verunreinigungen entfernen. Um die Reaktionsprodukte aus der Reaktionskammer zu entfernen, ist eine Unterdruckquelle über eine Rohrleitung mit der Reaktionskammer verbunden, die einen Abfluss der Reaktionsprodukte aus der Reaktionskammer bewirkt.

Es ist Aufgabe der vorliegenden Erfindung, eine verbesserte Vorrichtung zur Reinigung von Gegenständen, insbesondere von Matratzen zu schaffen, die auch eine Tiefenreinigung ermöglicht.

Diese Aufgabe wird mit einer Vorrichtung zur Reinigung von Gegenständen mit den Merkmalen des Anspruchs 1 gelöst.
Erfindungsgemäß umfasst die Vorrichtung zur Reinigung von Gegenständen eine Einrichtung zum Ansaugen von Umgebungsluft, eine Einrichtung zur Erzeugung von Plasma und Mittel zum Ausblasen einer Mischung von Umgebungsluft und Plasma in den zu reinigenden Gegenstand. Dadurch kann die Mischung von Umgebungsluft und Plasma den Gegenstand nicht nur oberflächlich reinigen, sondern wird in den Gegenstand eingeblasen, so dass die Reinigung auch mit einer gewissen Tiefenwirkung erfolgt. Das Plasma ist dabei vorzugsweise ozonhaltig, so dass auch Keime und Sporen sicher abgetötet werden können. Eine solche Reinigung ist besonders materialverträglich und beeinträchtigt nicht die Nutzung des Gegenstandes, beispielsweise aufgrund von Nässe. Zudem sind keine chemischen Verbrauchsmaterialien notwendig. Es ist ein Drucksensor vorgesehen, mittels dem ein Überdruck im Bereich der Auslassöffnung erfassbar ist. Ferner kann eine Steuerung vorgesehen sein, mittels der das Gebläse abhängig von dem erfassten Druck regelbar ist. Beispielsweise kann bei einem hohen Strömungswiderstand, was einen hohen Druck bewirkt, das Gebläse mit höherer Umdrehungszahl betrieben werden, um eine Tiefenreinigung bei dem Textil zu gewährleisten.

Gemäß einer bevorzugten Ausgestaltung der Erfindung ist ein Gehäuse vorgesehen, an dem mindestens eine Ausblasöffnung angeordnet ist, die allseitig von einer Dichtung umgeben ist. Das Gehäuse kann dabei an einer Unterseite eine oder mehrere Ausblasöffnungen aufweisen, die von der Dichtung umgeben sind, so dass ein gewisser Überdruck aufgebaut werden kann, was eine besonders gute Tiefenreinigung ermöglicht.

Die Einrichtung zur Erzeugung von Plasma umfasst vorzugsweise mindestens eine Elektrode, an der eine elektrische Entladung erzeugbar ist. Die Elektrode kann dabei geschützt in dem Gehäuse angeordnet sein, so dass kein Sicherheitsrisiko für den Benutzer gegeben ist. Eine entsprechende Spannungsversorgungseinheit mit einem Transformator kann ebenfalls in die Vorrichtung integriert sein.

In einer weiteren Ausgestaltung der Erfindung ist ein Gebläse zum Ansaugen von Umgebungsluft und Ausblasen der Mischung aus Umgebungsluft und Plasma vorgesehen. Das Gebläse kann dabei im Strömungsweg sowohl vor als auch hinter der Einrichtung zur Erzeugung von Plasma vorgesehen sein. Der Querschnitt des Strömungsweges kann im Bereich des Gebläses vergleichsweise klein gewählt werden, so dass eine hohe Strömungsgeschwindigkeit vorhanden ist, während der Querschnitt im Bereich der Einrichtung zur Erzeugung von Plasma größer gestaltet ist, um eine etwas langsamere Strömungsgeschwindigkeit zu erhalten.

In einer bevorzugten Ausgestaltung der Erfindung weist die Einrichtung zur Erzeugung von Plasma einen Formkörper auf, an dem mindestens eine Entladungselektrode und mindestens eine Einkopplungselektrode kapazitiv miteinander gekoppelt sind. Dadurch kann die Einrichtung zur Erzeugung von Plasma als Modul in ein Gehäuse eingebaut werden. Der Formkörper kann dabei eine Vielzahl von Luftkanälen aufweisen, durch die Umgebungsluft strömt, so dass das erzeugte Plasma effektiv durchmischt und weitertransportiert werden kann.

Um ein Austreten der Mischung von Plasma und Umgebungsluft zu einer Seite hin zu vermeiden, kann die Dichtung als elastischer Dichtgummi ausgebildet sein, der eine seitliche Abdichtung mit dem Textil bildet, so dass ein gewisser Überdruck zum Einblasen aufrecht erhalten wird.

Die Einrichtung zur Erzeugung von Plasma weist bevorzugt eine Vielzahl von Piezo-Elementen auf, die für eine kompakte Bauweise sorgen.

Für eine einfache Handhabung der Vorrichtung kann an dem Gehäuse ein Handgriff zur Führung vorgesehen sein, so dass die Vorrichtung leicht entlang des zu reinigenden Gegenstandes bewegt werden kann.

Zusätzlich vorteilhaft ist die Kopplung der Plasmareinigung mit einer mechanischen Reinigungsvorrichtung. Gerade in Bereichen starker Keimbelastung, etwa bei der Fugenreinigung oder bei Schimmelbildung an Wänden, lassen sich Oberflächen dadurch effizienter reinigen. Die mechanische Reinigungsvorrichtung kann dabei in Form einer Bürste, eines Schwamms oder eines Scheuerpads am Boden der Vorrichtung ausgebildet sein, welcher auf einer zu reinigenden Oberfläche aufliegt.

Da bei der Reinigung von engen Zwischenräumen und Fugenflächen oft kein Platz ist, um eine mechanische Reinigung der Oberfläche zusätzlich zur Plasmareinigung zu ermöglichen, sieht die Erfindung in einer bevorzugten Ausführung einen mechanischen Antrieb vor, welcher die Reinigungsvorrichtung vorzugsweise in rotierenden oder oszillierenden Bewegungen antreibt.

Um ein optimales Einwirken und eine gewisse Eindringtiefe zu gewährleisten, weist das Material der Bürste, des Schwamms oder des Scheuerpads gasdurchlässige Poren auf, wodurch die Plasmateilchen an die zu reinigende Oberfläche geleitet werden.

Für eine Reinigung von stark verschmutzen Oberflächen auf einem begrenzten Bereich, beispielsweise bei Fugen, ist eine rohrförmige Ausbildung des Gehäuses vorteilhaft. Die Abmessungen des Gehäuses befinden sich bevorzugt in einem Verhältnis von Länge zu Breite von 3:1 bis 20:1.

Die Erfindung wird nachfolgend anhand mehrerer Ausführungsbeispiele mit Bezug auf die beigefügten Zeichnungen näher erläutert. Sie zeigen:

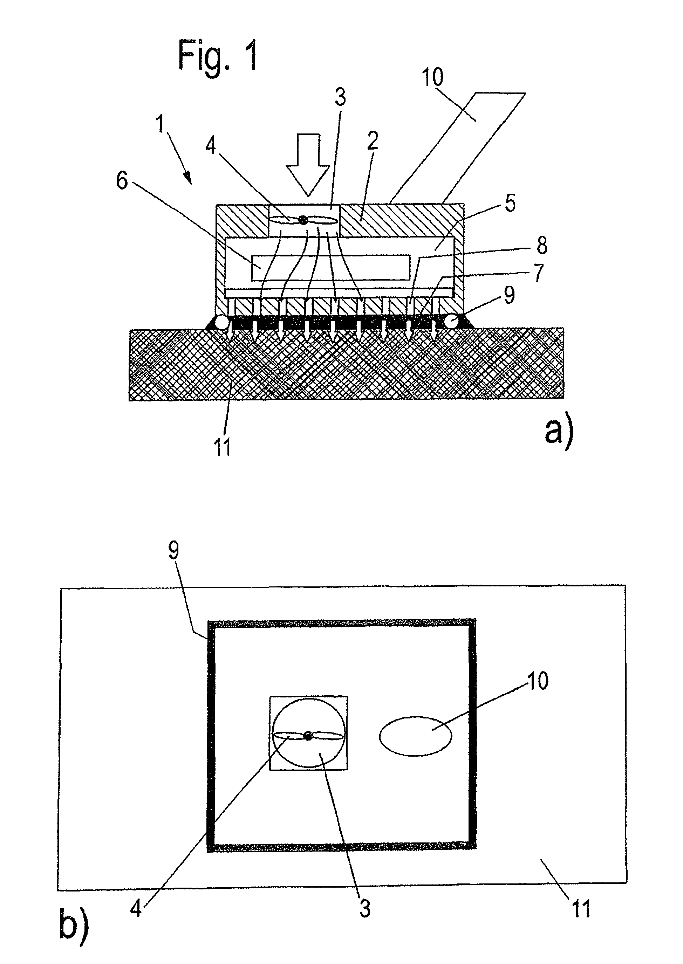

- Figur 1A, B: zwei Ansichten eines ersten Ausführungsbeispiels einer erfindungsgemäßen Vorrichtung zur Reinigung von Gegenständen;
- Figur 2: eine geschnittene Seitenansicht der Vorrichtung der Figuren 1A und 1B,
- Figur 3: eine geschnittene Seitenansicht einer modifizierten Vorrichtung zur Reinigung von Gegenständen,
- Figur 4 a), b): zwei geschnittene Seitenansichten von weiteren erfindungsgemäßen Vorrichtungen zur Reinigung von Gegenständen, und
- Figur 5 a) - c): drei geschnittene Seitenansichten von modifizierten Vorrichtungen zur Reinigung von Gegenständen.

Eine Vorrichtung 1 zur Reinigung von Gegenständen umfasst ein Gehäuse 2, an dem eine Einsaugöffnung 3 zum Ansaugen von Umgebungsluft vorgesehen ist. Ferner ist ein schematisch dargestelltes Gebläse 4 vorgesehen, um die Umgebungsluft anzusaugen und in eine Kammer 5 zu blasen, in der eine Einrichtung 6 zur Erzeugung von Plasma angeordnet ist. Die eingesaugte Luft vermischt sich mit dem hergestellten ozonhaltigen Plasma in der Kammer 5 und wird dann an eine Unterseite des Gehäuses 2 durch eine Vielzahl von Ausblasöffnungen 8 in einem Boden 7 des Gehäuses 2 ausgeblasen. Unterhalb des Bodens 7 ist ein Raum ausgebildet, der randseitig durch eine umlaufende Dichtung 9 in Form eines elastischen Gummiprofils begrenzt ist. Dadurch wird der Raum unterhalb des Bodens 7 abgedichtet, so dass die ausgeblasene Mischung aus Umgebungsluft und Plasma in den zu reinigenden Gegenstand 11 mit Überdruck eingeblasen werden kann. Der Gegenstand 11 kann insbesondere als Matratze, Teppich, Polstermöbel etc. ausgebildet sein.

Um das Gehäuse 2 entlang dem zu reinigenden Gegenstand 11 führen zu können, ist ein hervorstehender Handgriff 10 vorgesehen, der eine leichte Handhabung gewährleistet. Über den Handgriff 10 kann das Gehäuse 2 mit der Dichtung 9 gegen den zu reinigenden Gegenstand 11 gedrückt werden, um eine Abdichtung zu erreichen.

An dem Gehäuse 2 kann ferner ein Drucksensor vorgesehen sein, mittels dem ein Überdruck im Bereich der Ausblasöffnungen 8 unterhalb des Bodens 7 erfassbar ist. Dieser Drucksensor ist mit einer Steuerung gekoppelt, die das Gebläse 4 steuert. Wenn ein hoher Überdruck festgestellt wird, also der Strömungswiderstand durch den Gegenstand 11 groß ist, kann das Gebläse 4 stärker betrieben werden, um eine Tiefenreinigung an dem Gegenstand 11 zu gewährleisten.

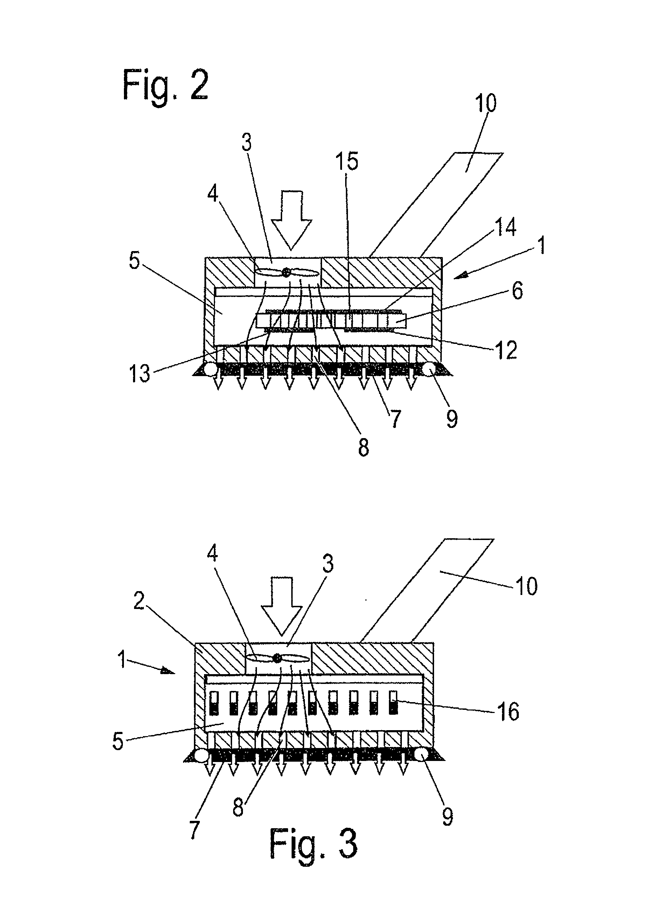

In Figur 2 ist die Vorrichtung 1 im Schnitt dargestellt, wobei in der Kammer 5 die Einrichtung 6 zur Erzeugung von Plasma dargestellt ist, die einen Formkörper umfasst, der an einer Seite zwei Einkopplungselektroden 12 und 13 besitzt, die kapazitiv mit einer Entladungselektrode 14 gekoppelt sind, die an der gegenüberliegenden Seite des Formkörpers angeordnet ist. Die Elektroden 12 und 13 sind mit einer Spannungsversorgungseinheit verbunden, die bei einer geeigneten Spannung und Frequenz für eine Entladung an der Entladungselektrode 14 sorgt, so dass sich ozonhaltiges Plasma ausbildet, das sich in der Kammer 5 mit der eingesaugten Luft durchmischt. Es ist auch möglich, mehrere der Formkörper bzw. mehrere Elektroden 12, 13, 14 vorzusehen, um die Menge an Plasma zu erhöhen. In dem Formkörper sind ferner Luftkanäle 15 ausgebildet, so dass sich die Luft besonders effektiv mit dem erzeugten Plasma durchmischt. Anschließend tritt die Mischung aus Umgebungsluft und Plasma über bodenseitig angeordnete Luftkanäle 8 aus der Kammer 5 aus.

In Figur 3 ist eine modifizierte Ausführungsform einer Vorrichtung 1' dargestellt, bei der im Gehäuse 2 eine Kammer 5 ausgebildet ist, in der eine Vielzahl von Piezo-Elementen 6' angeordnet sind, die zur Erzeugung von Plasma dienen. Die eingesaugte Luft wird somit durch das Gebläse 4 in die Kammer 5 eingeblasen und tritt dann am Boden des Gehäuses 2 aus. Die Anzahl der Piezo-Elemente 6' ist an den Volumenstrom der eingesaugten Luft angepasst.

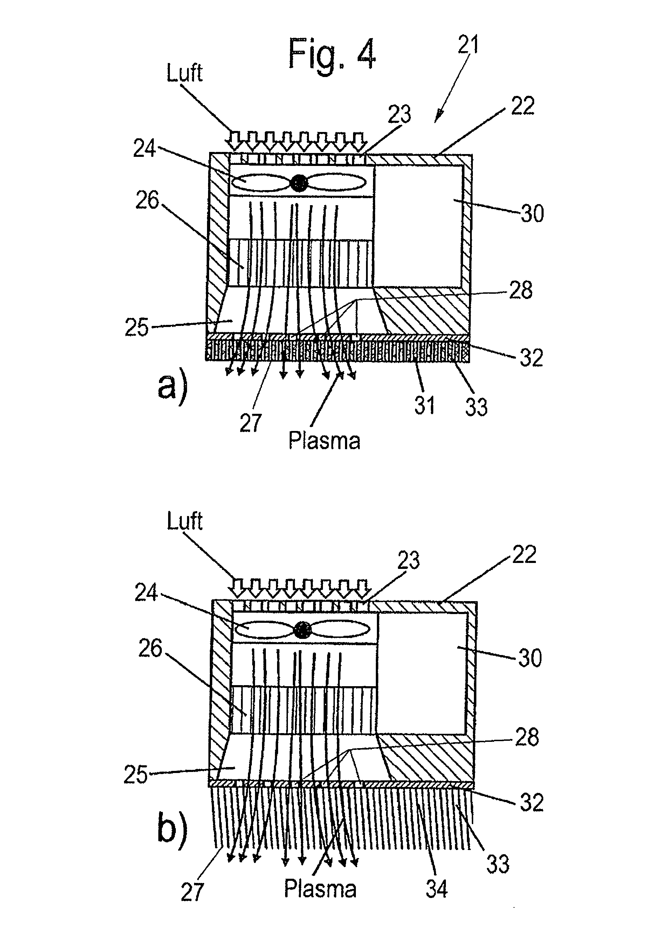

Fig. 4a zeigt eine modifizierte Vorrichtung 21, welche die Reinigung durch Plasmaerzeugung mit einer mechanischen Reinigung kombiniert. Die Vorrichtung 21 verfügt über eine, in einem Gehäuse 22 angebrachte Ansaugöffnung 23 und ein Gebläse 24, welches für die kontinuierliche Luftzufuhr von Umgebungsluft und Plasmaabfuhr sorgt und eine erhöhte Konzentration von Plasma vermeidet.

Dabei ist durch eine Steuerung des Gebläses 24 die Strömungsgeschwindigkeit der angesaugten Umgebungsluft ins Gehäuse einstellbar. Die Luft wird in einer Kammer 25 an der Einrichtung 26 zur Plasmaerzeugung in ein ozonhaltiges Plasma/Luftgemisch umgewandelt. Die Einrichtung zur Plasmaerzeugung 26 ist mit einem internen Stromspeichern 30 gekoppelt. Eine externe Stromversorgungs/- speichereinheit ist ebenfalls möglich.

Das Luft/Plasmagemisch tritt entlang einer Unterseite 27 der Vorrichtung 21 aus dem Gehäuse 22 aus. Ein Scheuerpad 31, das durch eine Klebeschicht oder ein Klettband 32 am Gehäuse 22 befestigt ist, kann zur Reinigung einer Oberfläche von abgetötetem biologischem Material eingesetzt werden. Dabei weist das Scheuerpad 31 Gaskanäle 33 auf, die mit dem Plasma/Luftgemisch durchströmt werden.

Fig. 4b zeigt eine weitere Variante der Vorrichtung mit einer mechanischen Reinigungsvorrichtung. Alle Bauteile entsprechen in ihrer Ausführungsart der Beschreibung in Figur 4a, bis auf die mechanische Reinigungsvorrichtung in Form einer Bürste 34. Sie ist durch eine Klebeschicht oder ein Klettband 32 am Gehäuse 22 befestigt. Die Borsten der Bürste verfügen über Gaskanäle 33.

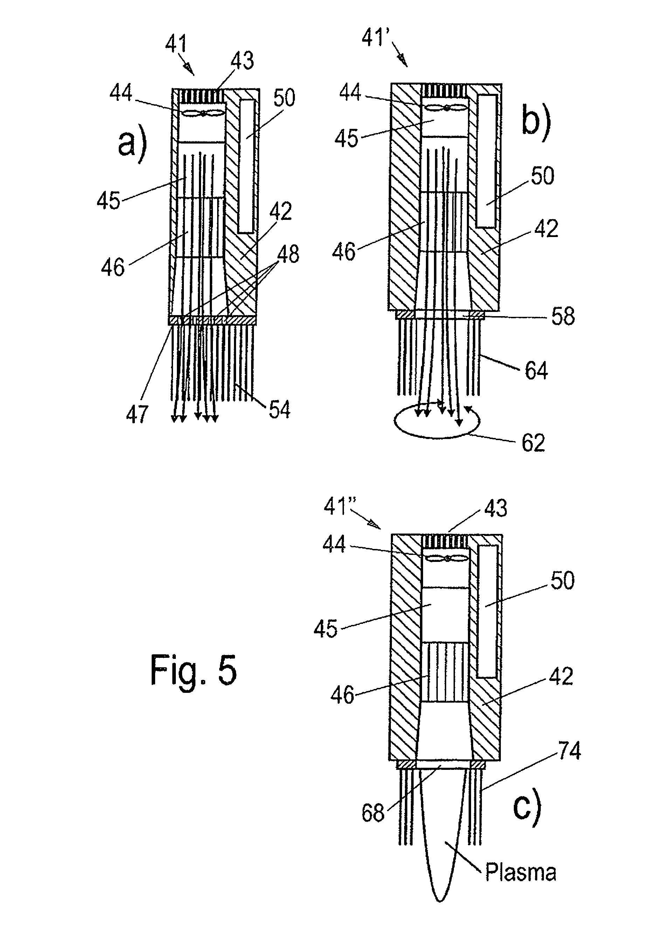

Figur 5a zeigt eine erfindungsgemäße Vorrichtung 41, bei welcher ein Gehäuse 42 rohrförmig ausgebildet ist und sich dadurch besonders zur Reinigung von Fugen und Ecken eignet. Über eine Ansaugöffnung 43 wird durch ein Gebläse 44 Umgebungsluft in eine Kammer 45 angesaugt. Die dabei erzeugte Strömungsgeschwindigkeit wird durch eine Steuerung des Gebläses 44 geregelt. Eine Einrichtung 46 zur Erzeugung von Plasma wandelt die Umgebungsluft anschließend teilweise in Plasma um. An der Unterseite 47 des Gehäuses 42 sind Ausblasöffnungen 48 angebracht, über die ein Austausch des Luft/Plasmagemisches mit einer zu reinigenden Oberfläche erfolgt.

An der Unterseite 47 des Gehäuses 42 ist eine Bürste 54 angebracht. Das Bürste verfügt dabei über Borsten mit Gaskanälen 53. Die Stromversorgung erfolgt über Stromspeicher 50, welcher im Gehäuse 42 der Vorrichtung integriert ist.

Eine spezielle Modifikation der Vorrichtung 41' der Figur 5a ist in Figur 5b dargestellt. Dabei ist schematisch eine Bürste 64 an der Peripherie der Ausblasöffnung 58 gezeigt, die rotierende oder oszillierende Bewegungen entsprechend dem Pfeil 62 ausführt. Die Austrittsöffnung 58 ist breiter als im vorhergehenden Beispiel um einen verbesserten Ausstoß des Plasma/Luftgemisches zu ermöglichen.

Figur 5c zeigt ebenso eine Modifikation der Vorrichtung 41" aus Figur 5a, wobei hierbei eine Bürste 74 statisch an der Peripherie der Ausblasöffnung 68 angebracht ist. Die Breite der Ausblasöffnung 68 ermöglicht die Ausbildung eines laminaren Strömungsprofils des austretenden Plasma/Luftgemisches und damit eine Bündelung des Plasmastroms.

### Bezugszeichen

- Vorrichtung: 1, 21, 41
- Gehäuse: 2, 22, 42
- Ansaugöffnung: 3,23,43
- Gebläse: 4, 24, 44,
- Kammer: 5, 25, 45
- Einrichtung: 6, 6', 26, 46
- Boden: 7,
- Unterseite: 27,47
- Ausblasöffnung: 8, 28, 48, 58, 68
- Dichtung: 9
- Handgriff: 10
- Gegenstand: 11
- Einkopplungselektrode: 12, 13
- Entladungselektrode: 14
- Luftkanal: 15
- Piezo-Element: 16
- Stromversorgung: 30, 50
- Scheuerpad: 31
- Klebe- oder Klettband: 32
- Gaskanäle: 33,53
- Bürste: 34, 54, 64, 74
- Pfeil: 62

## Patentansprüche

1. Vorrichtung zur Reinigung von Gegenständen umfassend:
- eine Einrichtung (4, 24, 44) zum Ansaugen von Umgebungsluft,
- eine Einrichtung (6, 6', 26, 46) zur Erzeugung von Plasma und
- Mittel zum Ausblasen einer Mischung von Umgebungsluft und Plasma in den zu reinigenden Gegenstand (11), **dadurch gekennzeichnet, dass** die Vorrichtung einen Drucksensor umfasst, mittels dem ein Überdruck im Bereich einer Ausblasöffnung (8, 28, 48, 58, 68) erfassbar ist.

2. Vorrichtung nach Anspruch 1, **dadurch gekennzeichnet, dass** ein Gehäuse (2) vorgesehen ist, an dem mindestens eine Ausblasöffnung (8) angeordnet ist, die allseitig von einer Dichtung (9) umgeben ist.

3. Vorrichtung nach Anspruch 1 oder 2, **dadurch gekennzeichnet, dass** die Einrichtung zur Erzeugung von Plasma mindestens eine Elektrode (14, 6') umfasst, an der eine elektrische Entladung erzeugbar ist.

4. Vorrichtung nach einem der Ansprüche 1 bis 3, **dadurch gekennzeichnet, dass** ein Gebläse (4, 24, 44) zum Ansaugen von Umgebungsluft und Ausblasen der Mischung aus Umgebungsluft und Plasma vorgesehen ist.

5. Vorrichtung nach einem der Ansprüche 1 bis 5, **dadurch gekennzeichnet, dass** eine Steuerung vorgesehen ist, mittels der das Gebläse abhängig vom erfassten Druck regelbar ist.

6. Vorrichtung nach einem der Ansprüche 1 bis 5, **dadurch gekennzeichnet, dass** die Einrichtung zur Erzeugung von Plasma einen Formkörper (6) aufweist, an dem mindestens eine Entladungselektrode (14) und mindestens zwei Einkopplungselektroden (12, 13) kapazitiv miteinander gekoppelt sind.

7. Vorrichtung nach Anspruch 6, **dadurch gekennzeichnet, dass** an dem Formkörper (6) eine Vielzahl von Luftkanälen (15) vorgesehen ist, durch die mit Plasma angereicherte Luft strömt.

8. Vorrichtung nach einem der Ansprüche 1 bis 7, **dadurch gekennzeichnet, dass** die Dichtung (9) als elastischer Dichtgummi ausgebildet ist.

9. Vorrichtung nach einem der Ansprüche 1 bis 8, **dadurch gekennzeichnet, dass** die Einrichtung zur Erzeugung von Plasma eine Vielzahl von Piezo-Elementen (6') aufweist.

10. Vorrichtung nach einem der Ansprüche 1 bis 9, d**adurch gekennzeichnet, dass** an einem Gehäuse (2) ein Handgriff (10) zur Führung der Vorrichtung entlang des zu reinigenden Gegenstandes vorgesehen ist.

11. Vorrichtung nach Anspruch 1 bis 10, **dadurch gekennzeichnet, dass** an einer Unterseite (27, 47) des Gehäuses (22, 42) eine Reinigungsvorrichtung zur mechanischen Reinigung in Form einer Bürste (34, 54, 64, 74) und/oder eines Schwamms und/oder eines Scheuerpads (31) vorgesehen ist.

12. Vorrichtung nach dem Anspruch 11, **dadurch gekennzeichnet, dass** die Reinigungsvorrichtung über einen Antrieb (62) bewegbar ist.

13. Vorrichtung nach dem Anspruch 11 oder 12, **dadurch gekennzeichnet, dass** das Material der Reinigungsvorrichtung gasdurchlässige Poren oder Kanäle (33, 53) aufweist.

14. Vorrichtung nach einem der Ansprüche 1 bis 13, **dadurch gekennzeichnet, dass** das Gehäuse (42) rohrförmig mit einem Verhältnis von Länge zu Breite von 3:1 bis 20:1 ausgebildet ist.

## Claims

1. A device for cleaning objects, comprising:
- a device (4, 24, 44) for drawing in ambient air,
- a device (6, 6', 26, 46) for generating plasma and
- means for blowing out a mixture of the ambient air and plasma into the object (11) to be cleaned, **characterised in that** the device comprises a pressure sensor, by means of which an excess pressure in the area of a blowing-out opening (8, 28, 48, 58, 68) can be detected.

2. The device according to claim 1, **characterised in that** a housing (2) is provided, on which at least one blowing-out opening (8) is disposed, which opening is surrounded on all sides by a seal (9).

3. The device according to claim 1 or 2, **characterised in that** the device for generating plasma comprises at least one electrode (14, 6') at which an electrical discharge can be generated.

4. The device according to any one of claims 1 to 3, **characterised in that** a blower (4, 24, 44) is provided for drawing in ambient air and blowing out the mixture of ambient air and plasma.

5. The device according to any one of claims 1 to 5, **characterised in that** a control unit is provided, by which means the blower can be regulated depending on the detected pressure.

6. The device according to any one of claims 1 to 5, **characterised in that** the device for generating plasma comprises a moulded body (6), on which at least one discharge electrode (14) and at least two coupling electrodes (12, 13) are capacitively coupled to one another.

7. The device according to claim 6, **characterised in that** a plurality of air channels (15) is provided on the moulded body (6), through which air enriched with plasma flows.

8. The device according to any one of claims 1 to 7, **characterised in that** the seal (9) is configured as an elastic sealing rubber.

9. The device according to any one of claims 1 to 8, **characterised in that** the device for generating plasma comprises a plurality of piezoelectric elements (6').

10. The device according to any one of claims 1 to 9, **characterised in that** a handle (10) for guiding the device along the object to the cleaned is provided on the housing (2).

11. The device according to any one of claims 1 to 10, **characterised in that** a cleaning device for mechanical cleaning in the form of a brush (34, 54, 64, 74) and/or a sponge and/or a scouring pad (31) is provided on an underside (27, 47) of the housing (22, 42).

12. The device according to claim 11, **characterised in that** the cleaning device can be moved by means of a drive (62).

13. The device according to claim 11 or 12, **characterised in that** the material of the cleaning device has gas-permeable pores or channels (33, 53).

14. The device according to any one of claims 1 to 13, **characterised in that** the housing (42) is configured to be tubular with a length-to-width ratio of 3:1 to 20:1.

## Revendications

1. Dispositif pour nettoyer des objets comprenant :
- un système (4, 24, 44) pour aspirer de l'air ambiant,
- un système (6, 6', 26, 46) pour générer du plasma et
- des moyens pour évacuer par soufflage un mélange d'air ambiant et de plasma dans l'objet (11) à nettoyer, **caractérisé en ce que** le dispositif comprend un capteur de pression, au moyen duquel une surpression dans la zone d'un dispositif de soufflage (8, 28, 48, 58, 68) peut être détectée.

2. Dispositif selon à revendication 1, **caractérisé en ce qu'**il est prévu un boîtier (2), sur lequel est disposée au moins une ouverture de soufflage (8), qui est entourée de tous côtés par un joint (9).

3. Dispositif selon la revendication 1 ou 2, **caractérisé en ce que** le système pour générer du plasma comporte au moins une électrode (14, 6'), sur laquelle une décharge électrique peut être générée.

4. Dispositif selon l'une des revendications 1 à 3, **caractérisé en ce qu'**une soufflante (4, 24, 44) est prévue pour aspirer de l'air ambiant et évacuer par soufflage le mélange de l'air ambiant et du plasma.

5. Dispositif selon l'une des revendications 1 à 5, **caractérisé en ce qu'**il est prévu une commande, au moyen de laquelle la soufflante peut être régulée en fonction de la pression enregistrée.

6. Dispositif selon l'une des revendications 1 à 5, **caractérisé en ce que** le système pour générer du plasma présente un corps moulé (6), sur lequel au moins une électrode de décharge (14) et au moins deux électrodes d'injection (12, 13) sont couplées l'une à l'autre de façon capacitive.

7. Dispositif selon la revendication 6, **caractérisé en ce qu'**une pluralité de canaux d'air (15), par lesquels de l'air enrichi avec du plasma circule, est prévue sur le corps moulé (6).

8. Dispositif selon l'une des revendications 1 à 7, **caractérisé en ce que** le joint (9) est conçu comme caoutchouc d'étanchéité élastique.

9. Dispositif selon l'une des revendications 1 à 8, **caractérisé en ce que** le système pour générer du plasma présente une pluralité d'éléments piézoélectriques (6').

10. Dispositif selon l'une des revendications 1 à 9, **caractérisé en ce qu'**une manette (10) est prévue sur un boîtier (2) pour le guidage du dispositif le long de l'objet à nettoyer.

11. Dispositif selon les revendications 1 à 10, **caractérisé en ce qu'**un dispositif de nettoyage est prévu sur un côté inférieur (27, 47) du boîtier (22, 42) pour le nettoyage mécanique sous forme d'une brosse (34, 54, 64, 74) et/ou d'une éponge et/ou d'un tampon abrasif (31).

12. Dispositif selon la revendication 11, **caractérisé en ce que** le dispositif de nettoyage peut être déplacé au moyen d'un entraînement (62).

13. Dispositif selon la revendication 11 ou 12, **caractérisé en ce que** le matériau du dispositif de nettoyage présente des pores ou des canaux (33, 53) perméables au gaz.

14. Dispositif selon l'une des revendications 1 à 13, **caractérisé en ce que** le boîtier (42) est conçu en forme de tuyau avec un rapport longueur/largeur de 3:1 jusqu'à 20:1.
